# EUROPEAN PATENT SPECIFICATION

(11) **EP 4 522 047 B1**
(45) Date of publication and mention of the grant of the patent: **11.11.2026**
(21) Application number: 23728959.0
(22) Date of filing: 10.05.2023
(51) Int. Cl.: A61B 17/32

(54) **ASPIRATION FLUE FOR ULTRASONIC SURGICAL HORN**
RAUCHROHR FÜR EIN CHIRURGISCHES ULTRASCHALLHORN
GAINE D'ASPIRATION POUR CORNET CHIRURGICAL À ULTRASONS

(30) Priority: 10.05.2022 US 202263340329 P
(43) Date of publication of application: 19.03.2025
(60) Divisional application: 26204915.8 / 4 830 650
(73) Proprietor: Integra LifeSciences Enterprises, LLLP, Princeton, NJ 08540 (US)
(72) Inventor: SHEEHAN, James, Princeton, New Jersey 08540 (US); ALLEN, Patrick, Princeton, New Jersey 08540 (US); COTTER, Daniel J., North Easton, Massachusetts 02356 (US)
(74) Representative: Gill Jennings & Every LLP
(86) International application number: PCT/US2023/021735
(87) International publication number: WO 2023/220177

(56) References cited:
- AU-A1- 2020 213 767
- US-A1- 2019 159 793

## Description

### BACKGROUND OF THE INVENTION

The present invention relates generally to ultrasonic surgical devices, and more particularly, to ultrasonic surgical aspirators for removing diseased tissues.

Devices that effectively utilize ultrasonic energy for a variety of applications are well known in a number of diverse arts. One of these devices is an ultrasonic horn or tip used for the removal of tissue. The Ampulla or Gaussian profile was published by Kleesattel as early as 1962, and is employed as a basis for many ultrasonic horns in surgical applications including devices for use in ultrasonic aspiration as described in U.S. Pat. No. 4,063,557 to Wuchinich, et al, 1977, and U.S. Pat. No. 6,214,017 to Stoddard, et al, 2001. The Gaussian profile is used in practice to establish and control the resonance and mechanical gain of horns. A resonator, a connecting body, and the horn act together as a three-body system to provide a mechanical gain, which is defined as the ratio of output stroke amplitude of the distal end of the tip to the input amplitude of the resonator. The mechanical gain is the result of the strain induced in the materials of which the resonator, the connecting body, and the ultrasonic horn are composed.

A magnetostrictive or piezoelectric transducer coupled with the connecting body functions as a first stage of the booster horn with a mechanical gain of about 2:1, due to the reduction in area ratio of the wall of the complex geometry. The major diameter of the horn transitions to the large diameter of the Gaussian segment in a stepped-horn geometry with a gain of as large as about 5:1, again due to reduction in area ratio. The uniform strain along the length of the Gaussian segment provides multiplicative gain of typically less than 2:1. Thus, the application of ultrasonically vibrating surgical devices used to fragment and remove unwanted tissue with significant precision and safety has led to the development of a number of valuable surgical procedures.

Certain devices known in the art characteristically produce continuous vibrations having substantially constant amplitude at a frequency of about 20 to about 55 kHz, for example, at a predetermined frequency of 20-36 kHz. Amplitude of transducer-surgical tip systems decreases with increasing frequency because maximum stress in the material of the horns is proportional to amplitude times frequency, and the material must be maintained to an allowed fraction of its yield strength to support rated life in view of material fatigue limits. For example, U.S. Pat. Nos. 4,063,557, 4,223,676 and 4,425,115. disclose devices suitable for the removal of soft tissue which are particularly adapted for removing highly compliant elastic tissue mixed with blood. Such devices are adapted to be continuously operated when the surgeon wishes to fragment and remove tissue, and generally is operated by a foot switch.

Ultrasonic aspiration has become the standard of care for removal of tumors and diseased tissue in neurosurgery and general surgery. Typically, ultrasonic surgical aspirators for fragmenting and aspirating tissue include an ultrasonic transducer supported within a handpiece, an ultrasonically vibrating horn or tip operably connected to the ultrasonic transducer, and a sleeve or flue positioned about the horn. The horn includes a longitudinally extending central bore having one end located adjacent a distal tip and a second end located adjacent the proximal end of the horn. The proximal end of the horn is adapted to engage a vacuum source to facilitate aspiration of fluid. The flue is positioned about the horn to define an annular passage. Irrigation fluid is supplied through the annular passage around the horn to the surgical site where it mixes with blood and tissue particles and is aspirated through the bore in the horn. By mixing the irrigation fluid with the blood and tissue particles, coagulation of the blood is slowed down and aspiration thereof is aided. U.S. Pat. Nos. 5,015,227 and 4,988,334 disclose such ultrasonic surgical devices. For example, a titanium surgical tip may be powered by a transducer to fragment tissue and suction effluent via a central channel. A flue is employed to deliver irrigation liquid, usually saline, and it protects tissue along the path to the surgical site from the vibrating surgical tip. The transducer vibrates along its length, and ultrasonic horns such as stepped horns and specialty profiles of reduced diameter amplify vibration.

A known instrument on the market for the ultrasonic fragmentation of tissue at an operation site and aspiration of the tissue particles and fluid away from the site is the CUSA^{®} Excel Ultrasonic Surgical Aspirator (Integra LifeSciences Corporation, Plainsboro, N.J.). When the longitudinally vibrating tip in such an aspirator is brought into contact with tissue, it gently, selectively, and precisely fragments and removes the tissue. The CUSA transducer amplitude can be adjusted independently of the frequency and this amplitude can be maintained under load depending on reserve power of the transducer. In simple harmonic motion devices, the frequency is independent of amplitude. Advantages of this unique surgical instrument include minimal damage to healthy tissue in a tumor removal procedure, skeletoning of blood vessels, prompt healing of tissue, minimal heating or tearing of margins of surrounding tissue, minimal pulling of healthy tissue, and excellent tactile feedback for selectively controlled tissue fragmentation and removal.

In an apparatus that fragments tissue by the ultrasonic vibration of a tool tip, efficiency of energy utilization is optimized when the transducer which provides the ultrasonic vibration operates at resonant frequency. The transducer and surgical tip design establishes the resonant frequency of the system, while the generator tracks the resonant frequency and produces the electrical driving signal to vibrate the transducer at the resonant frequency. However, changes in operational parameters, such as changes in temperature, thermal expansion, and load impedance, result in deviations in the resonant frequency. Accordingly, controlled changes in the frequency of the driving signal are required to track the resonant frequency. This is controlled automatically in the generator.

Conventional ultrasonic surgical aspirating tips employed in surgery for many years typically present a longitudinally vibrating annular surface with a central channel providing suction or aspiration, which contacts tissue and enables fragmentation via described mechanisms of mechanical impact (momentum), cavitation, and ultrasound propagation. Mechanical impact may be most useful in soft tissue and cavitation clearly contributes to the fragmentation of tenacious and hard tissue in situations where liquids are present and high intensity ultrasound exceeds the cavitation threshold. When tissue ablation surgery is performed, the ultrasonic surgical aspirator may be inserted into the patient through a trocar. Once inserted, the surgeon may fragment and aspirate tissue at the surgical site using the activated ultrasonic tip. The flue may deliver irrigation to the surgical site in a laparoscopic surgery application. The flue may serve as a protective barrier between the active ultrasonic tip and the trocar and/or human body.

Ultrasound propagation is concerned with the transmission of pressure across the boundary of a surgical tip and tissue, which leads to the propagation of pressure and, perhaps more importantly, particle displacement. Acoustic impedance is the total reaction of a medium to acoustic transmission through it, represented by the complex ratio of the pressure to the effective flux, that is, particle velocity times surface area through the medium. As discussed in the classic text of Krautkramer J. and Krautkramer H, ULTRASONIC TESTING OF MATERIALS, Berlin, Heidelberg, N.Y., 1983, for the case of a low to high acoustic impedance boundary, it may seem paradoxical that pressure transmitted can exceed 100%, but that is what results from the build-up of pressure from a low to high acoustic impedance boundary. In the case of a high to low acoustic impedance mismatch, such as with a high impedance titanium ultrasonic horn to low impedance fibrous muscle, soft tissue, or water, the pressure transmitted decreases (e.g., less than 15% for titanium to fibrous muscle) and particle displacement increases (e.g., as great as 186% for titanium to muscle).

Typical all-silicone flues may have longitudinal deflection occur during movement through a trocar as well as visually obscures the working surface of the ultrasonic tip. The silicone rubber has a high coefficient of friction, low stiffness, and drags in the trocar, resulting in substantial total deflection when this friction force is applied to a long length of silicone.

Hence, those skilled in the art have recognized a need to reduce friction, increase stiffness, reduce stretching due to the trocar, and/or maintain the distal end visible throughout surgical manipulation. The present invention fulfills this need and others. AU2020213767 A1, US2019/159793 A1 and US2022/087699 A1 disclose devices of the prior art.

### SUMMARY OF THE DISCLOSURE

Claim 1 and 8 define the invention and dependent claims disclose embodiments. No surgical methods are claimed per se. In some embodiments of the disclosure for example, a flue for use with an ultrasonic horn may include a base having a first connector and a second connector interconnected by a base body. In various embodiments, the base body may include a first end and an opposing second end. In some embodiments, the first end of the base body may include the first connector and the second end of the base body may include the second connector. In some embodiments, the first connector may include a first overmold portion adapted to engage a nosecone. In various embodiments, the second connector may include a second overmold portion.

In addition, in some embodiments, at least one of the first connector and the second connector may be secured to the base body by at least one of a plastic weld, an adhesive bond, and an adhesive seal. In various embodiments, at least one of the first connector and the second connector may be secured to the base body by the plastic weld and the adhesive bond. In some embodiments, at least one of the first connector and the second connector may be secured to the base body by the adhesive seal. In various embodiments, at least one of the first connector and the second connector may include one or more ribs, and wherein the one or more ribs are overmolded by the first overmold portion and the second overmold portion, respectively. In embodiments, the first overmold portion defines at least a portion of an irrigation port. In various embodiments, the second overmold portion may include a through opening narrowing away from the second connector.

In some embodiments, an ultrasonic surgical apparatus for fragmenting tissue and removing fragmented tissue may include a surgical handpiece comprising a housing, a nosecone attached to the housing, a flue attached to the nosecone, and/or a transducer mounted within the housing. In various embodiments, the apparatus may include a surgical tip connected to the transducer via an internal horn. In some embodiments, the apparatus may include an irrigation system connected to the handpiece for supplying irrigation fluid adjacent the surgical site for suspending fragmented tissue. In various embodiments, the apparatus may include an aspirating system connected to the handpiece for aspirating fluid and tissue fragmented at the surgical site. In some embodiments, the flue may have a first end and an opposing second end. In various embodiments, the flue may include a base, a first overmold portion defining the first end, and/or a second overmold portion defining the second end.

In addition, in some embodiments, the base may include a base body having a first end and an opposing second end, a first connector, and/or a second connector. In various embodiments, the first connector may be connected to the first end of the base body and the second connector may be connected to the second end of the base body. In some embodiments, the first connector may include the first overmold portion and the second connector may include the second overmold portion. In various embodiments, at least one of the first connector and the second connector may include one or more ribs engaging the first overmold portion and the second overmold portion, respectively. In some embodiments, at least one of the first connector and the second connector may be secured to the first end and the second end of the base body, respectively, by at least one of a plastic weld, an adhesive bond, and/or an adhesive seal. In various embodiments, at least one of the first connector and the second connector may be secured to the first end and the second end of the base body, respectively, by the plastic weld and the adhesive bond. In some embodiments, at least one of the first connector and the second connector may be secured to the first end and the second end of the base body, respectively, by the adhesive seal. In various embodiments, the first connector and the second connector may be made of a material different from the first overmold portion and the second overmold portion. In some embodiments, the base body may be made of a material different from the first and second overmold portions. In some embodiments the proximal seal could be eliminated and, instead, a complete sealed rigid (e.g., polycarbonate etc.) section included. Overall, the embodiments constitute a composite or hybrid flue (overmolded proximal sealing material such as silicone to rigid portions such as polycarbonates to distal overmolded sealing and arc resistant material such as silicone) that supports ultrasonic energy and radio frequency (RF) energy propagation and transmission necessary to fragment and coagulate tissue in surgery. The combination of the hybrid flue and extendable surgical tip supports the longest ultrasonic aspirator instruments in the field.

In some embodiments, a method for attaching members of an ultrasonic surgical apparatus may include providing a nosecone. In various embodiments, the method may include providing a first flue having a first length. In some embodiments, the first flue may include one or more overmold portions on a first connector and a second connector, and wherein the first connector and the second connector is attached to each end of a first base body. In some embodiments, the method may include providing a handpiece having a body. In various embodiments, the method may include providing a tip. In some embodiments, the method may include connecting the nosecone to the handpiece.

In addition, in various embodiments, the method may include providing a second flue having a second length different from the first length, wherein the second flue includes one or more overmold portions on a first connector and a second connector, and wherein the first connector and the second connector is attached to each end of a second base body. In some embodiments, the method may include connecting at least one of the first flue and the second flue to the nosecone. In various embodiments, the method may include connecting the first flue to the nosecone.

In some embodiments, a method for attaching members of a flue may include overmolding a first overmold portion onto a first connector. In various embodiments, the method may include overmolding a second overmold portion onto a second connector. In some embodiments, the method may include providing a base body having a first end and a second end. In various embodiments, the method may include connecting the first connector and first overmold portion to the first end of the base body. In some embodiments, the method may include connecting the second connector and the second overmold portion to the second end of the base body.

In addition, in various embodiments, the method may include connecting the first connector and the second connector to the base body by at least one of a plastic weld, an adhesive bond, and/or an adhesive seal. In some embodiments, the method may include connecting the first connector and the second connector to the base body by the plastic weld and the adhesive bond. In various embodiments, the step of overmolding the first overmold portion and the second overmold portion on the first connector and the second connector, respectively, may occur before the steps of connecting the first connector and the second connector to the base body. In some embodiments, the first overmold portion and the second overmold portion are overmolded on one or more ribs projecting outwardly from an outer periphery of the first connector and the second connector, respectively. In various embodiments, each of the first connector and the first overmold portion may define at least a portion of an irrigation port. In some embodiments, the method may include varying a length of the base body from the first end to the second end to vary a length of the flue.

In some embodiments, an ultrasonic horn may include a first horn member, a second horn member, and/or one or more third horn members connecting the first horn member to the second horn member for a predetermined overall length of the horn.

In addition, in some embodiments, at least one of the third horn members may be a half-wavelength of about 107 mm. In various embodiments, the third horn member may be positioned at an antinode. In some embodiments, the method may include a threaded coupling between the third horn member and each one of the first horn member and the second horn member.

In some embodiments, a method of varying the length of an ultrasonic horn may include the step of providing a first horn member. In various embodiments, the method may include providing a second horn member. In some embodiments, the method may include determining an overall length of the ultrasonic horn. In some embodiments, the method may include selecting one or more third horn members to achieve the overall length of the ultrasonic horn. In various embodiments, the method may include coupling the one or more third horn members between the first horn member and the second horn member. Such flue and extendable surgical tip can result in the longest ultrasonic aspirator surgical instruments in the field.

In addition, in some embodiments, the coupling may be a threaded engagement. In various embodiments, the method may include fastening the one or more third horn members pneumatically with specialized equipment. In various embodiments, the method may include over torqueing the coupling. In some embodiments, the coupling may be adjacent an antinode.

Other features and advantages of the present invention will become more apparent from the following detailed description of the disclosure when taken in conjunction with the accompanying exemplary drawings.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Embodiments of the presently disclosed ultrasonic horn are described herein with reference to the drawings, in which:
FIG. 1 is a perspective view of an ultrasonic apparatus in accordance with the present invention;
FIG. 2 illustrates the proximal end of the apparatus of FIG. 1 in more detail;
FIG. 3 is a perspective view a nosecone fully assembled to a handpiece/nosecone and supporting the flue (the flue tube is not shown in this drawing);
FIG. 4A is a perspective view of one embodiment of an ultrasonic horn;
FIG. 4B is an exploded view of the ultrasonic horn of FIG. 4A;
FIG. 4C is a side sectional view of the ultrasonic horn of FIG. 4A;
FIG. 4D is a perspective view of another embodiment of an ultrasonic horn illustrating a third horn member increasing the length of the horn as compared to the horn shown in FIG. 4A;
FIG. 4E is an exploded view of the ultrasonic horn of FIG. 4D;
FIG. 4F is a side sectional view of the ultrasonic horn of FIG. 4D;
FIG. 4G is a perspective view of another embodiment of an ultrasonic horn illustrating two third horn members increasing the length of the horn as compared to the horn shown in FIG. 4D;
FIG. 4H is an exploded view of the ultrasonic horn of FIG. 4G;
FIG. 4I is a side sectional view of the ultrasonic horn of FIG. 4G;
FIG. 5 is a sectional view of an embodiment of a flue in accordance with the present invention (the flue tube is not shown in this drawing);
FIG. 6A shows a perspective view of an embodiment of a first connector;
FIG. 6B shows another perspective view of the first connector of FIG. 6A;
FIG. 6C shows a perspective view of an embodiment of a second connector;
FIG. 6D shows another perspective view of the second connector of FIG. 6C;
FIG. 7A shows a perspective view of the first connector of FIG. 6A combined with an embodiment of a first overmold portion;
FIG. 7B shows a perspective view of the second connector of FIG. 6C combined with an embodiment of a second overmold portion;
FIGS. 8A-8F illustrate multiple views of the first connector of FIG. 6A;
FIGS. 9A-9F illustrate multiple views of the second connector of FIG. 6C;
FIG. 10 is an enlarged sectional view of one embodiment of the connection between the second connector and the base body;
FIG. 11 is an enlarged sectional view of one embodiment of the connection between the first connector and the base body.

### DETAILED DESCRIPTION OF THE PREFERRED EMBODIMENTS

Embodiments of the presently disclosed ultrasonic horn will now be described in detail with reference to the drawings, in which like reference numerals designate identical or corresponding elements in each of the several views. As used herein, the term "distal" refers to that portion of the instrument, or component thereof which is farther from the user while the term "proximal" refers to that portion of the instrument or component thereof which is closer to the user during normal use. The terms "ultrasonic horn," "ultrasonic tip," "ultrasonic aspirating tip," "ultrasonic surgical aspirating tip," "aspirating tip," "ultrasonic surgical tip," "surgical tip" and "tip" are used herein interchangeably. The type of energy capable of being used is discussed primarily as "ultrasonic", but can also include radio frequency (RF) energy. The terms "flue," "irrigation flue," "sleeve," "irrigation manifold" and "manifold" are used herein interchangeably. The terms "tip extender" and "horn extender" are used herein interchangeably.

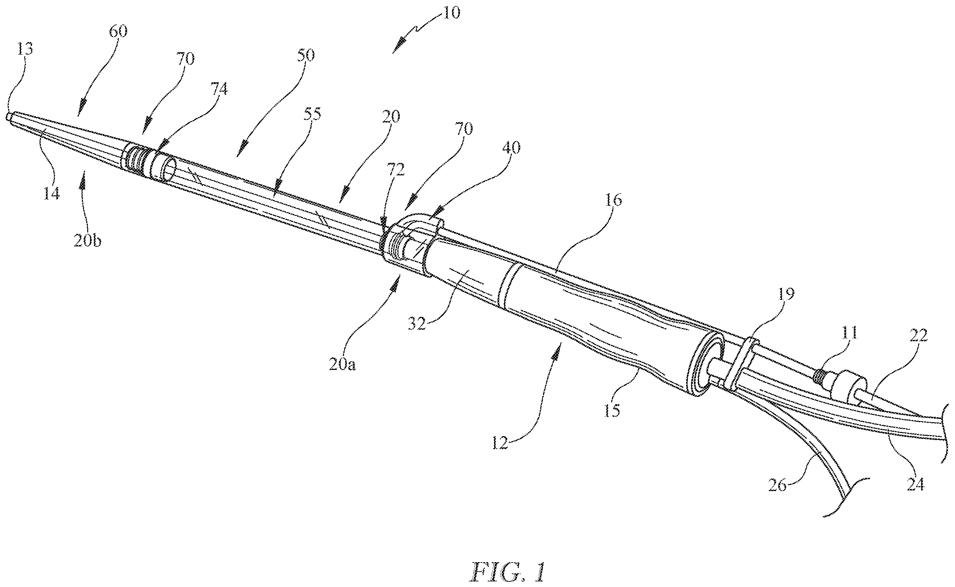

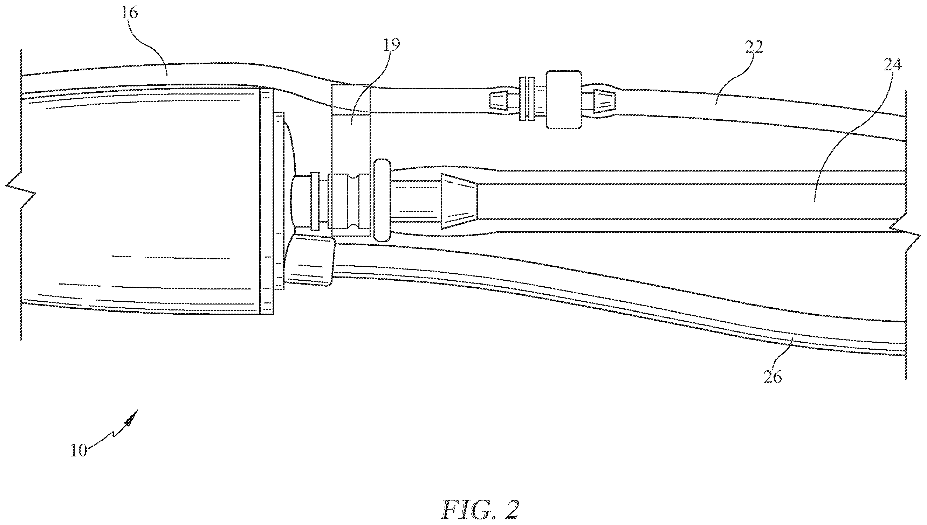

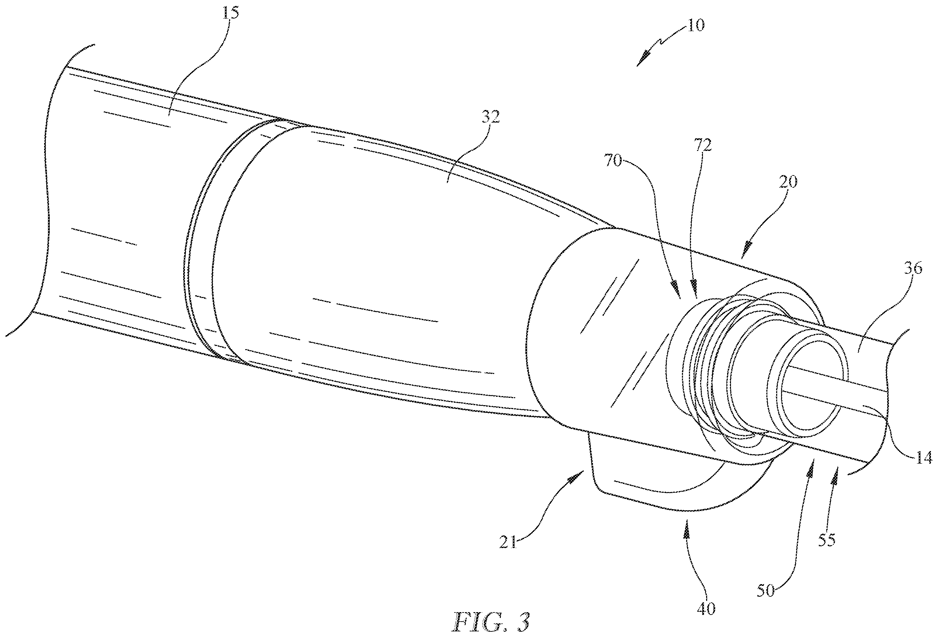

Referring now to FIGS. 1-3, one embodiment of the presently disclosed apparatus for ultrasonically fragmenting and aspirating tissue is shown. The present disclosure is directed to an ultrasonic surgical apparatus 10 for ultrasonically fragmenting and aspirating tissue in a surgical operation. Generally the ultrasonic surgical apparatus includes a handpiece 12 used by a surgeon to direct fragmentation. The handpiece 12 encases a transducer (not shown) on which a surgical tip or ultrasonic horn 14 is fastened. The ultrasonic horn can be powered by the transducer and be ultrasonically actuated to fragment tissue and suction effluent via a central channel. A distal end 13, or portions thereof, of the ultrasonic horn 14 extends beyond a distal end of the flue 20. The ultrasonic horn 14 is vibrated to fragment tissue during surgery. The ultrasonic horn may be made of titanium or other conventional materials known in the art.

A cooling and irrigation system which provides cooling fluid to the ultrasonic horn 14 is provided for maintaining temperature within an acceptable range. The handpiece 12 includes a housing 15 which may be formed of a sterilizable plastic or metal, but is preferably plastic. The flue 20 provides a path for irrigation fluid or liquid and connects to the distal end of the housing 15. The flue 20 typically interfaces to the handpiece 12 via a nosecone 32. The flue 20 may include or attach to a flue tube 16 and be in fluid communication with the flue tube 16 through an opening 21. The nosecone 32 attaches to the handpiece 12 and covers the internal portion of the ultrasonic horn 14.

An irrigation tube 22 connects to the flue tube 16 up-stream and supplies irrigation fluid through the flue tube 16 to an operative site during surgery. An aspiration tube 24 provides suction and a path for aspiration from the operative site to a collection canister (not shown). Alternatively, the aspiration tube may be mounted externally of the housing 15. A flue tube clip 19 allows for adjustment of the location of the flue tube 16 per that favored by the surgeon during operation. Also shown is an electrical cable 26 for providing power to the apparatus or providing switching connections.

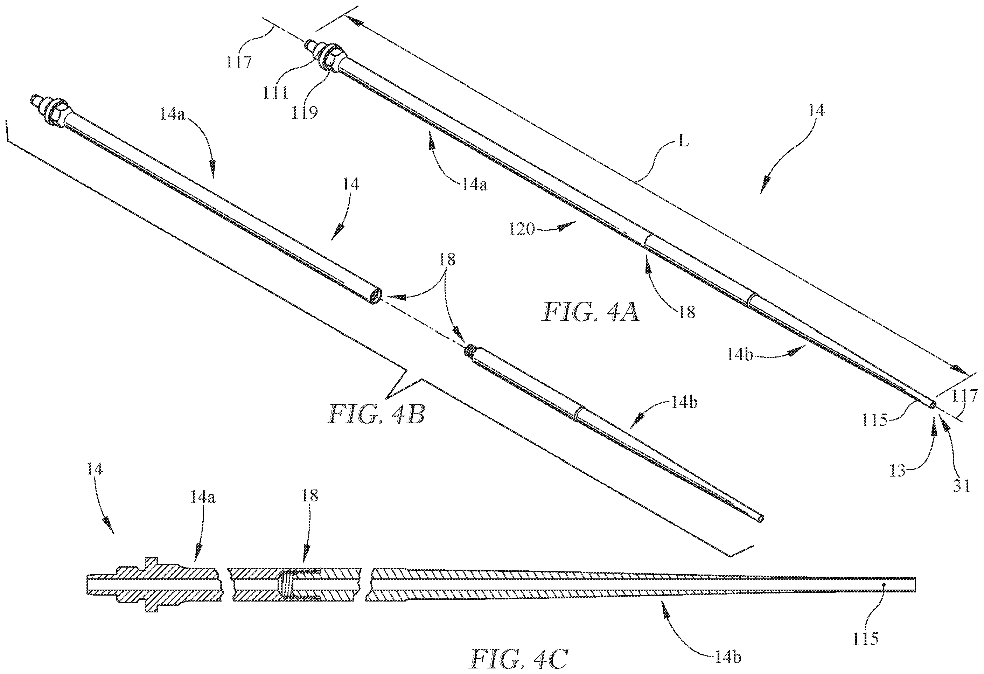

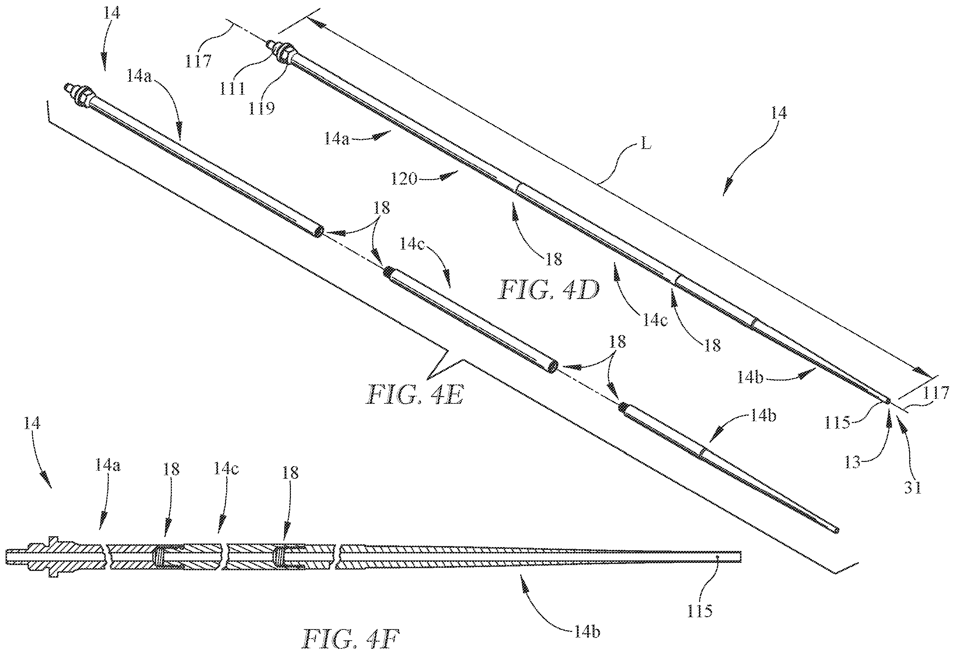

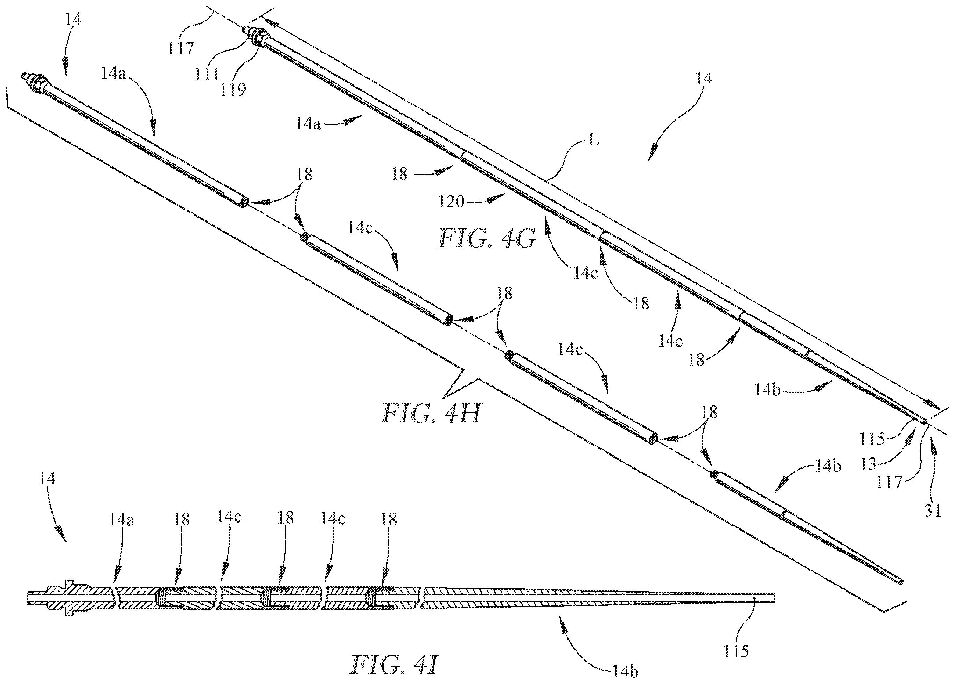

FIGS. 4A-4C illustrates one embodiment of an ultrasonic horn 14, which is suitable for use with the above-described ultrasonic surgical apparatus for fragmenting and aspirating tissue. The ultrasonic horn has an external surface 120 and includes a first horn or proximal member 14a and a second horn or distal member 14b extending distally from the first horn or proximal member. Although the first horn member 14a is coupled/connected to the second horn member 14b by a threaded coupling 18 (e.g. male-female connection), it should be understood that a variety of connections/couplings or methods of combining two or more horn members (e.g. 14a, 14b, 14c, etc.) may be used. For example, the threaded coupling 18, if used, may be over torqued and/or laser welded. In some embodiments, the one or more couplings of the horn members may be a press fit. The press fit, if used, may be laser welded and/or an electron beam weld connection. Another example of the press fit may be secured with a pin. In the one embodiment shown, the first horn member 14a may include a female or tapped thread on a distal end and the second horn member 14b includes a male thread on a proximal end to define the threaded coupling 18. A shown in FIGS. 4D-4I, the ultrasonic horn 14 may have one or more additional horns, modules, or members 14c (e.g. third horn member 14c) to vary the length (e.g. overall) of the horn for one or more applications. In the one embodiment shown, the third horn member(s) 14c may include a female or tapped thread on a distal end and a male thread on a proximal end to define the threaded coupling 18, or portions thereof. The ultrasonic horn 14 has a distal end portion 13 and a threaded proximal end 111, a throughbore 117, a preaspiration hole or transverse bore 115, and a hexagon engagement portion 119. The ultrasonic horn may have a larger external diameter in the first horn or proximal member 14a section and a smaller external diameter in the second horn or distal member 14b section.

Although the ultrasonic horn as shown is not stepped, it is known that there are ultrasonic horns that are stepped. In some embodiments not shown, the ultrasonic horns can have a single long horn body, rather than two or more horns/members of two or more different diameters. A single long horn extender can have a constant external diameter throughout its length or have a gradually changing diameter along its length, for example, gradually decreasing in diameter along its length distally. In addition, one or more horns/members may form steps or transition smoothly from another horn/member without forming any apparent step. The ultrasonic horn may vibrate in the ultrasonic frequency range with a longitudinal amplitude in excess of about 5 mils (0.005 inch) to 14 mils (0.014 inch).

The throughbore 117 may also have a larger diameter section within the first horn 14a and a smaller diameter section within the second horn 14b. The diameters of the proximal larger diameter of first horn, the distal smaller diameter portions of the second horn, or diameters of one or more third members, if used, of the throughbore may have any suitable diameters as can be readily determined as appropriate by those skilled in the art. For example, the distal smaller diameter throughbore portion may be about 0.078 inches (1.9812 mm) in diameter. The throughbore does not necessarily have to correspond to the geometry of the one or more members/horns. The throughbore may have two or more diameters in a stepped fashion or otherwise, a constant diameter throughout its length, or a gradually changing (for example, decreasing) diameter along its length distally.

The ultrasonic horn 14 is substantially circular in cross section and disposed within the flue 20. During operation of the ultrasonic apparatus 10, irrigation fluid is supplied through the opening 21 into the flue 20. Flue 20 and the ultrasonic horn 14 define an annular cavity 36 therebetween. Irrigation fluid is supplied from flue 20 through cavity 36 to the distal end of the ultrasonic horn 14. A transverse bore is formed in preaspiration holes 115 near the distal end of the ultrasonic horn 14 and communicates with the throughbore 117. The irrigation fluid is drawn from preaspiration holes 115 and the surgical site into inlet 31 of the throughbore 117 along with fragmented tissue, blood, etc., and is removed from the surgical site via the throughbore 117 and the aspiration tube 24. The transverse bore provides an alternate route for fluid to enter throughbore 117 when inlet 31 becomes clogged or occluded by tissue intended for removal. The preaspiration holes 115 ensure a substantial amount of irrigation is available in a continuous cooling circuit. The irrigation also aids in preventing or reducing immediate clotting of blood that could clog the channel or become occluded by tissue intended for removal. The preaspiration holes 115 ensure a substantial amount of irrigation is available in a continuous cooling circuit. The irrigation also aids in preventing or reducing immediate clotting of blood that could clog the channel.

In a more detailed aspect, irrigation liquid, for example saline, is necessary to cool the surgical tip and site of tissue fragmentation. This irrigation liquid is provided to the flue with a peristaltic pump at a rate as low as 2 to 3 ml/min, which is typically only about a drip or two a second. The irrigation liquid is supplied at the proximal end of the ultrasonic horn. The irrigation liquid progresses to near the distal end of the ultrasonic horn, where two preaspiration holes of 0.015 inch (0.381 mm) diameter suction a majority, perhaps 90-95%, of the irrigation through the holes connecting the outside horn diameter to the central suction channel. This action of irrigation and suction supports a contiguous cooling circuit for the vibrating titanium metal and it also helps to wet effluent such as blood and tissue in the central channel. Some irrigation is also favorable to cooling the surgical site, improving coupling to tissue, and affording cavitation necessary to emulsification and aspiration of tissue, such as tumors.

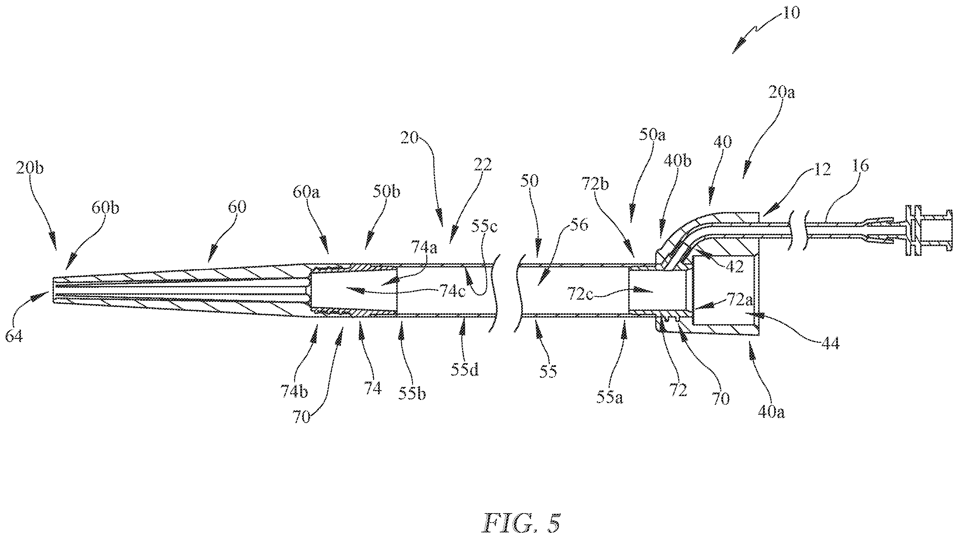

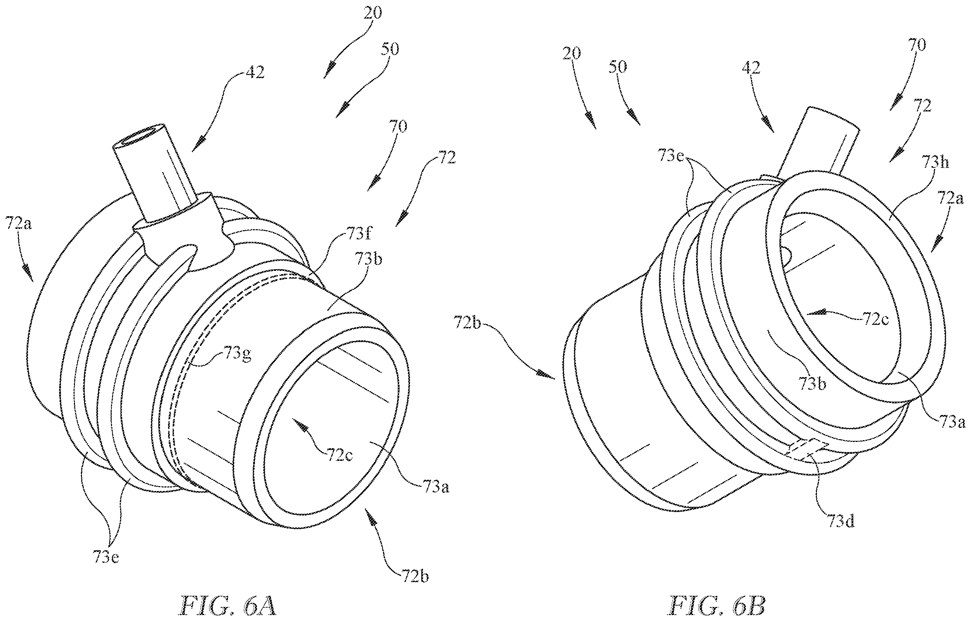

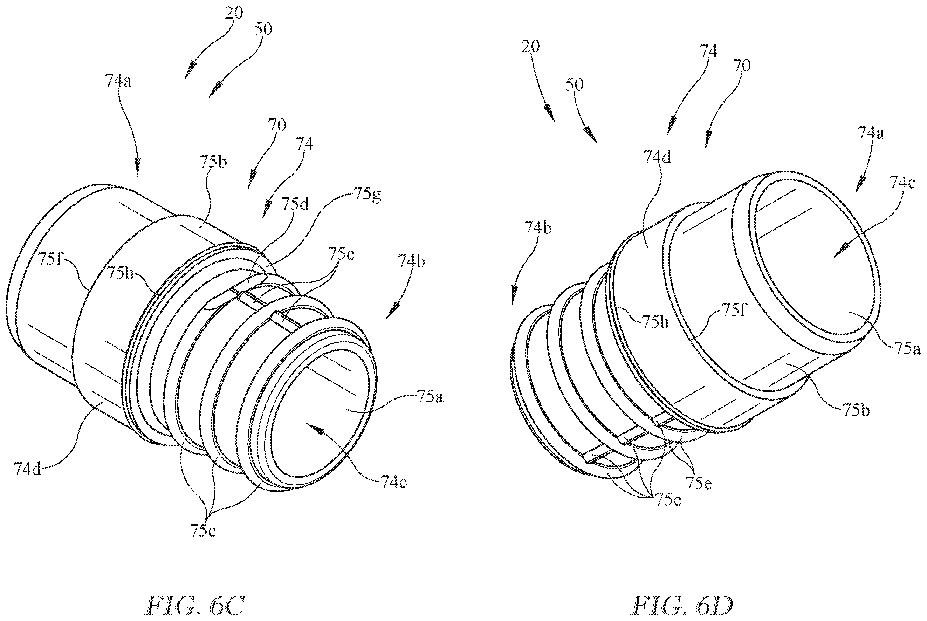

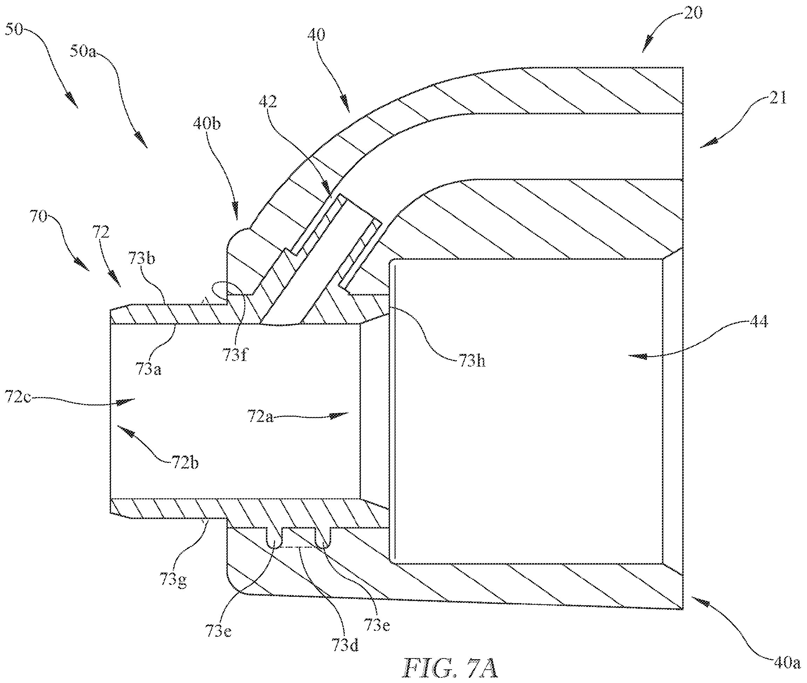

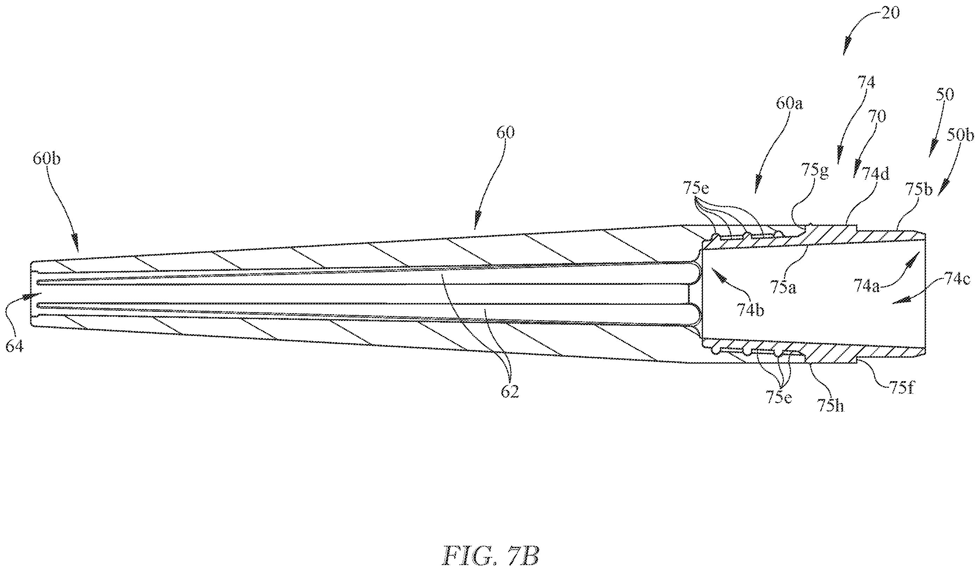

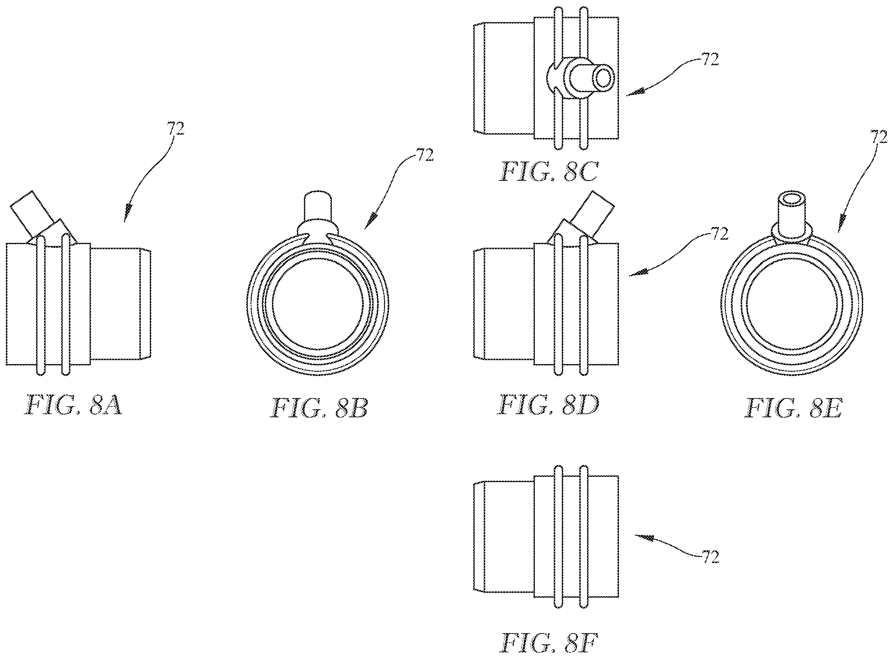

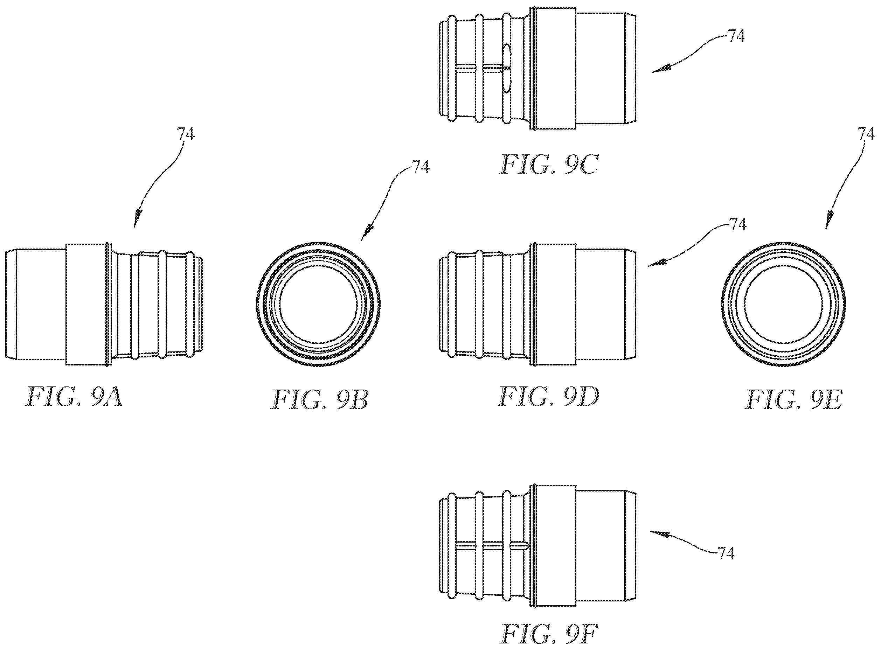

In some implementations, two or more components and/or shots of material may be overmolded together to manufacture a flue. The ultrasonic surgical apparatus 10 of FIG. 1 illustrates an overmolded flue 20 extending from the nosecone 32, or portions of the apparatus, towards the end 13 of the surgical tip 14. As shown in FIGS. 1-3 and 5-12E, the flue 20 may include at least one overmold shot of material (e.g. same or different) to manufacture the flue. Flue 20 may include a base 50, or portions thereof, (e.g. base body 55, connectors 70) and one or more overmold portions 40, 60. The flue 20 and/or body 22 may include opposing ends, one end 20a adjacent the nosecone 32 and the other end or free end 20b adjacent the free end 13 of the surgical tip 14 (e.g. bone tip). In the one embodiment shown in FIGS. 5, 10, and 11, the overmold portions 40, 60 may be adjacent each end 20a, 20b of the flue 20, connectors 70 (e.g. 72, 74), and/or base 50 (e.g. 50a, 50b). The overmold portions may include a first overmold portion 40 and a second overmold portion 60. The first overmold portion or flue boot 40 may be adjacent a first end 50a of the base 50 and/or first end 20a of the flue/body 22. The first overmold portion 40 (e.g. second end 40b) may be overmolded onto the base 50, or portions thereof (e.g. 70, 72). The second overmold portion or flue tip 60 may be adjacent a second end 50b of the base 50 and/or second end 20b of the flue/body 22. The second overmold portion 60 (e.g. first end 60a) may be overmolded onto the base 50, or portions thereof (e.g. 70, 74). The first overmold portion 40 of the flue 20 may contain a molded irrigation port 42 and/or portion of the flue opening 21, etc. The second overmold portion 60 (e.g. second end 60b) of the flue 20 may narrow from the base 50 (e.g. base body 55, second connector 74) in a direction towards the open free end 20b of the flue surrounding or adjacent to the surgical tip end 13. In some embodiments, the base 50, or portions thereof, may narrow towards the overmold portion 60 of the flue 20 or flue free end 20b. In various embodiments, the base 50 (e.g. base body 55) may be of a constant diameter (e.g. inner periphery and/or outer periphery). The inner periphery 55c may define a through-opening 56. In some embodiments, the first overmold portion 40 and the second overmold portion 60 may be made of one or more materials (e.g. silicone, polycarbonate, Acetal, Nylon, Radel^{®}, and the like). The first overmold portion or flue boot 40 may be made of a first material. The second overmold portion or flue tip 60 may be made of a second material. The first and second materials may be the same, or may be different. In some embodiments, the first material of the first overmold portion and the second material of the second overmold portion may be made of the same or different material. For example, both the first and second materials may be made of silicone. Further, the first overmold portion and the second overmold portion may be made in a single overmold shot over the base 50, or portions thereof (e.g. base body 55 and/or connectors 70). Alternatively, the first overmold portion 40 and the second overmold portion 60 may be made or overmolded in two or more different shots with the (e.g. base body 55 and/or connectors 70). As shown in the one embodiment, the first overmold portion 40 and the second overmold portion 60 may be made or overmolded over their respective connector 70 (e.g. first connector 72, second connector 74) in one or more shots of material (e.g. silicone over polycarbonate)

In some implementations, the flue 20, base 50, base body 55 and/or apparatus 10 may include one or more connectors 70 interconnecting the first overmold portion 40 and/or second overmold portion 60 to the base body 55. In the one embodiment shown in FIGS. 5-11, the base 50 may include a base body 55, a first/proximal connector/coupling 72, and/or a second/distal connector/coupling 74. The base 50 and/or base body 55 may be made of, but is not limited to, a polycarbonate, Acetal, Nylon, Radel^{®}, and/or any other extrudable or fiber-filled polymer. Such rigid sections can be extruded in commonly known processes and can be extended as needed for different length surgical tips. In some embodiments, the base 50 and/or base body 55 may be an extruded tube. The connectors 70 (e.g. 72 and/or 74) may be made of, but is not limited to, a polycarbonate, Acetal, Nylon, Radel^{®}, and/or any other extrudable or fiber-filled polymer. The base body 55 and/or connectors 70 may have a stiffness that may eliminate or reduce longitudinal deflection of the apparatus 10/flue 20, or portions thereof (e.g. during movement through a trocar). In some embodiments, the base body 55 and/or connectors 70 may have a rigidity in the range of about 100,000 psi to about 350,000 psi ( 1psi = 6894.76 Pascal)

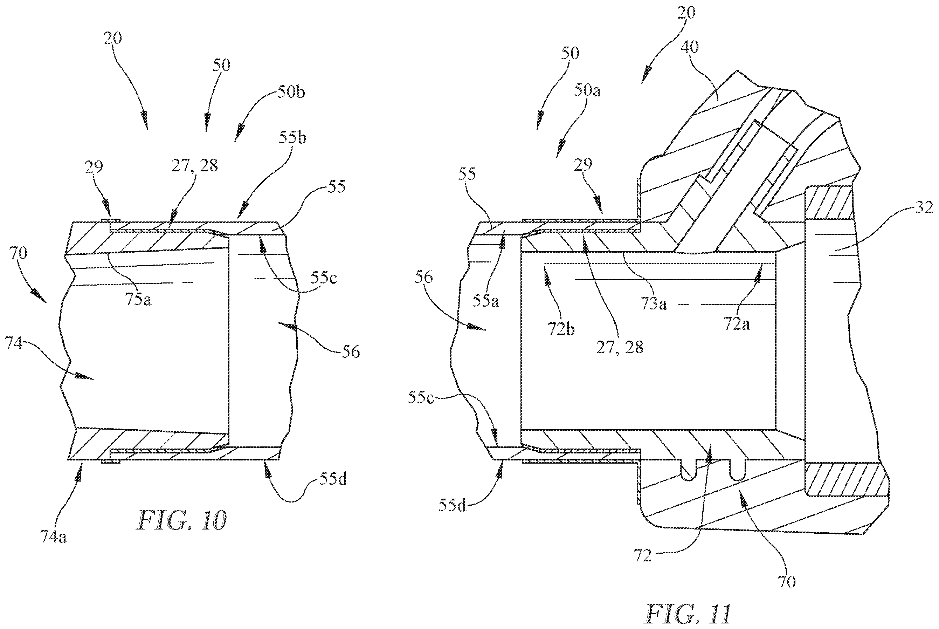

The base body 55 may include a first end 55a adjacent the first end 20a of the flue 20 and a second end 55b adjacent the second end 20b of the flue 20. The first connector 72, if used, may be adjacent the first end 55a of the base body 55. The second connector 74, if used, may be adjacent the second end 55b of the base body 55. The first overmold portion 40, if used, may be overmolded onto the first connector 72. The second overmold portion 60, if used, may be overmolded onto the second connector 74. For example, the silicone of the first overmold portion 40 and/or second overmold portion 60 may be molded over the polycarbonate of the first and second polycarbonate connector 72, 74, respectively. The one or more combined connectors 70 and overmold portions 40, 60 may be subsequently combined/fixed/secured to the opposing ends 55a, 55b of the base body 55, respectively. Although not shown, the connectors 70 may be attached to the base body 55 and then overmolded with one or more overmold portions 40, 60 (e.g. first overmold portion, second overmold portion, etc.). In some embodiments, the first overmold portion 40 and the first connector 72 and/or the second overmold portion 60 and the second connector 74 may be plastic/laser welded, adhesively bonded, and/or sealed (e.g. adhesive) to the base body 55 (e.g. first end 55a, second end 55b). One example of the adhesive to seal/bond the attachment between portions of the base may be, but is not limited to, LOCTITE brand adhesive. The one or more connectors 70 may be only plastic/laser welded to the one or more ends 55a, 55b of the base body 55. The one or more connectors 70 may be only adhesively bonded to the one or more ends 55a, 55b of the base body 55. The one or more connectors 70 may be plastic/laser welded and adhesively bonded to the one or more ends of the base body. The weld and/or adhesive bond may be circumferentially about the entire outer/inner peripheries (e.g. 360 degrees) as shown in the one embodiment or a portion thereof. Further, the adhesive seal, if used, may occur after the plastic/laser welding and/or adhesive bonding. The use of the adhesive sealant at the seam of the plastic welding and/or adhesive bond may be used as a secondary measure to seal inconsistencies, if any, in the laser/weld process thereby enhancing the dielectric resistance of this joint for electrosurgery safety. In some embodiments, the adhesive sealant may be applied to portions of the overmold portions. As shown in the one embodiment in FIGS. 10 and 11, a plastic/laser weld area/portion 27, an adhesive bond area/portion 28, and/or a seal area/portion 29 (e.g. adhesive) is used to connect one or more portions of the base body 55 and one or more portions of the connector(s)/overmold portions.

In some implementations, the base 50, flue 20, base body 55, and/or apparatus 10 may include at least first connector 72. As shown in FIGS. 5, 6A, 6B, 7A, and 8A-8F, the first connector 72 may include a first end 72a adjacent the first overmold portion 40 (e.g. second end 40b) and an opposing second end 72b adjacent the base body 55 (e.g. first end 55a). The first connector 72 may include an inner periphery 73a defining a through opening 72c. The first connector 72 may include an outer periphery 73b. The outer periphery 73b may include a decrease or step in diameter from the first end 72a towards the second end 72b. A gate 73d, if used, may be positioned on the outer periphery 73b (e.g. adjacent the one or more ribs 73e). The first connector 72 may contain/define a molded irrigation port 42 and/or portion of the opening 21, etc. As shown in the one embodiment, the port 42 may be positioned from the inner periphery 73a through the outer periphery 73b (e.g. larger diameter step) and projecting outwardly therefrom. The first connector 72 may include one or more annular ribs 73e projecting outwardly from the outer periphery 73b (e.g. larger diameter step). The ribs 73e may be continuous about the outer periphery as shown in the one embodiment. However, the ribs may not extend 360 degrees about the outer periphery in some embodiments. The ribs 73e may be longitudinally spaced from each other. The outer periphery 73b of the first end 72a (e.g. one or more annular ribs, port) of the first connector 72 may be overmolded by a portion of the first overmold portion 40. The first overmold portion 40 may project longitudinally towards the nosecone 32 from the first end 72a and/or radially outward from the outer periphery 73b. The first overmold portion 40 may cover the end face or radial step 73f facing towards the base body 55, between the larger diameter and smaller diameter outer periphery portions. There may be a crush rib 73g, if used, surrounding the outer periphery 73b of the smaller diameter outer periphery spaced from the step 73f adjacent the first overmold portion's longitudinal extent. The first connector 72 (e.g. second end 72b or smaller diameter outer periphery) may be inserted into the first end 55a of the base body 55 and secured (e.g. welded, adhesive bonded, and/or adhesively sealed, etc.). The first end 72a and an internal step extending radially outward from the outer periphery of the first connector may define an end face 73h. One or more portions of the end face 73h may engage/seal against the nosecone 32. The first overmold portion 40, or portions thereof, (e.g. inner periphery, longitudinal end face(s) facing towards the nosecone) may seal against one or more surfaces of the nosecone 32.

In some implementations, the base 50, flue 20, base body 55, and/or apparatus 10 may include at least one second connector 74. As shown in FIGS. 5, 6C, 6D, 7B, and 9A-9F, the second connector 74 may include a first end 74a adjacent the base body 55 (e.g. second end 55b) and an opposing second end 74b adjacent the second overmold portion 60 (e.g. first end 60a). The second connector 74 may include an inner periphery 75a defining a through opening 74c. The through opening 74c may taper from the first end 74a to the second end 74b. The second connector 74 may include an outer periphery 75b. Each end 74a, 74b of the second connector 74 may decrease in diameter away from the center or collar 74d. A gate 75d, if used, may be positioned on the outer periphery 75b (e.g. adjacent the one or more ribs 75e). The second connector 74 may include one or more annular ribs 75e projecting outwardly from the outer periphery 75b (e.g. second end 74b). The ribs 75e may be continuous about the outer periphery as shown in the one embodiment. However, the ribs may not extend 360 degrees about the outer periphery in some embodiments. The ribs 75e may be longitudinally spaced from each other. The outer periphery 75b of the second end 74b (e.g. one or more annular ribs) of the second connector 74 may be overmolded by a portion of the second overmold portion 60. The second overmold portion 60 may cover an end face 75f extending towards the second overmold portion 60 adjacent the second end 74b and/or end face or radial step 75g facing towards the second overmold portion 60 adjacent the larger diameter collar. There may be a crush rib 75h, if used, surrounding the outer periphery 75b of the collar adjacent the step 75g adjacent the second overmold portion's longitudinal extent. The second overmold portion 60 extends longitudinally away from the second end 74b towards the flue second end 20b or distal end 13. The second overmold portion 60 may include a plurality of longitudinal ribs or protrusions 62 projecting inwardly away from the inner periphery. The first end 74a or outer periphery 75b may be inserted into the second end 55b of the base body 55 and secured (e.g. welded, adhesive bonded, and/or adhesively sealed, etc.). The first end 74a and/or outer periphery 75b of the first end 74a and/or second overmold portion 60 may engage/seal against one or more surfaces of the second end 55b of the base body 55.

In some embodiments, the base 50, flue 20, and/or apparatus 10 may include a base body 55 having a first end 55a engaging the first connector 72 and/or first overmold portion 40 and a second end 55b engaging the second connector 74 and/or second overmold portion 60. As shown in FIGS. 5 and 11, the first end 55a may telescope with or overlap a portion of the first connector 72. The first end 55a may receive the second end 72b of the first connector 72. As shown in FIGS. 5 and 10, the second end 55b may telescope with or overlap a portion of the second connector 74. The second end 55b may receive the first end 74a of the second connector 74. The base body 55 may include an inner periphery 55c and an outer periphery 55d. The base body 55 may have a through opening 56 defined by the inner periphery 55c.

In some implementations, the boot or first overmold portion 40 may be overmolded over one or more portions/surfaces of the first connector 72. The first overmold portion may have a first end 40a and an opposing second end 40b. The first end 40a may engage the nosecone 32. The second end 40b may engage or overmold the first connector 72 (e.g. first end 72a, irrigation port 42). The first end 40a and/or second end 40b may define at least a portion of the irrigation port 42 and/or flue opening 21 alone or in combination with the first connector 72. The second end 40b may be overmolded onto one or more ribs 73e. A through opening 44 may extend through the first overmold portion 40.

In some implementations, the tip or second overmold portion 60 may be overmolded over one or more portions/surfaces of the second connector 74. The second overmold portion may have a first end 60a and an opposing second end 60b. The first end 60a may engage or overmold the second connector 74 (e.g. second end 74b). The second end 60b may surround the horn 14 and/or distal end 13. The second end 60b may include the one or more protrusions/ribs 62 (e.g. longitudinal, bumps, spheres, etc.). The first end 60a may be overmolded onto one or more ribs 75h. A through opening 64 may extend through the second overmold portion 60. The through opening 64 may narrow away from the second connector 74 or from the first end 60a towards the second end 60b.

In some embodiments, the flue 20, base 50, base body 55, and/or apparatus 10 may include a variety of lengths for a variety of applications of the surgical tip 14 having different lengths (e.g. one or more extensions). To adjust the length of the flue to accommodate a variety of surgical tip lengths, a plurality of base bodies 55, of varying lengths, may be manufactured and subsequently selected for an application length (e.g. first flue length, second flue length larger than the first flue length). For example, the base and/or base body may be extruded for a predetermined first length. The selected base body 55 having a predetermined first length is then overmolded and/or combined with the overmolded connectors 70 and 40/60 (e.g. combination connector and overmold portion). This may result in a first flue length for one application. In another application, the selected base body may be a predetermined second length. The selected base body 55 having a predetermined second length is then overmolded and/or combined with the overmolded connectors 70 and 40/60 (e.g. combination of connector and overmold portion). This may result in a second flue length, different from the first flue length, for another application.

It should be noted the configuration of using a silicone boot seal or first overmold portion 40 to a standard or electrosurgical nosecone supports high voltage breakdown strength and sealing necessary to prevent conductive saline from carrying potential to the surgeon or patient anatomy. Similarly, the surgical tip or second overmold portion 60 of silicone supports electrical safety and resistance to arcing and mechanical vibration of ultrasound. The distal silicone rubber or overmolded rubber is helpful to resist erosion and cracking due to arcing when, for example, RF is alied to the surgical tip for coagulation. Predicate devices and prototypes of completely rigid flues, without benefit of silicone, rapidly failed in testing due to arcing of electrosurgery.

In some implementations, the flue tip or second overmold portion 60 (e.g. silicone) may have a high melting point for durability with ultrasonic energy and electrosurgical arcing. Further, the flue tip may have a high dielectric resistance for limiting electrosurgical discharge to the working surface.

In some implementations, the flue boot or first overmold portion 40 (e.g. silicone) may have material compliance for friction fit with existing equipment (e.g. nosecones). Further, the flue boot may have a high dielectric resistance for limiting electrosurgical discharge to the working surface.

In some implementations, the flue body 22 or base body 55 (e.g. polycarbonate) may have reduced friction when inserted through a trocar, eliminating or reducing flue elongation and contraction from flue movement through the trocar (e.g. eliminates or reduces the visual obscuring of the tip). Further the extrusion length may be easily modified for different length laparoscopic tips by adjusting the length of the base body. Further, the flue body may have a high dielectric resistance for limiting electrosurgical discharge to the working surface.

In some implementations, overmolding the one or more overmold portions (e.g. first, second) on the connectors 70 instead of on opposing ends of the elongated extruded tube or base body may be advantageous. Silicone cure temperatures may approach or exceed the glass transition temperature of extrusion-grade polycarbonate. Overmolding to a separate insert/connector may allow that material to be chosen for injection moldability, which may be more compatible with preferred silicone cure temperatures. This also may simplify tooling, as the total overmold tool size only needs to accommodate the insert itself, rather than the entire length of the extruded tube or base body, also simplifying core pin shutoff on the inside diameter. This may also result in a more modular design which avoids the need for multiple sets overmold tools to produce multiple lengths of flue. It should be noted that care in selecting materials and opacity enables laser welding in some embodiments. The insert/connecter having a black polymer heats with absorption of the laser energy and in proximity to clear polymer (e.g. base body) causes both to heat and weld.

### Laparoscopic Surgical Tip

As shown in Figs. 4A-4I, one or more laparoscopic surgical tips 14 may have the first horn member 14a, the second horn member 14b, and the one or more third horn members 14c, if used, joined with one or more threaded couplings 18. The adjacent ends of the horn members define a portion of the couplings 18 to connect the horn members (e.g. 14a, 14b, 14c, etc.) to provide one or more predetermined lengths of one or more tips 14. In some implementations, this modular construction allows adequate straightness and concentricity of the gun-drilled central lumen over a limited length, to maintain uniform and minimal stress at the distal, tapering Gaussian region, in which material stresses are amplified to achieve substantial amplitude at the end.

Joining/coupling of the first horn member 14a, the second horn member 14b, and the one or more third horn members 14c, if used, may be done with a pneumatic vise or collet rather than standard flats for fastening with wrenches. This enables one or more horns 14 of one or more lengths for use in the operating room that may not be disassembled, either accidentally or deliberately (e.g., in an unauthorized attempt to modify the device). In some implementations as shown in FIGS. 4D-4I, the horn 14 may include one or more third horn members 14c for one or more lengths or applications. Each third horn member 14c may be approximately 90 mm to approximately 120 mm in length. For example, this could be 100 mm, which is the half- wavelength of the 23 kHz compressive standing wave in titanium. The one or more third horn members 14c, if used, may be a half-wavelength extender/member/module. The one or more third horn members 14a may have a different inner and/or outer diameter than at least one of the first horn member and second horn member in some embodiments. At 23 kHz the speed of sound in titanium produces a compressive wavelength (e.g. speed of sound divided by frequency) of approximately 214 mm, half of which is 107 mm, a practical length for the surgical extension. Thus, the extended length surgical tip having one or more third horn members may be long enough, even considering trocar placement, to cover the full range of liver resection. This was tested in a design validation with acceptable to highly acceptable geometry.

In some implementations, it was discovered that one or more half-wavelength extenders or third horn members 14c may be added and the Gaussian tapered section of the surgical tip, adjusted to obtain resonance and amplitude appropriate to surgery. Creating a modular system of tip subcomponents may allow these three or more subcomponents to create two different catalog items, by merely adding at least one third horn 14c to create the extended length tip. Although useful for efficiency of scale, this design approach may allow lateral modes, which are highly sensitive to diameter, to be suppressed specifically along the midsection of the extended length device. Increasing diameter moves lateral modes up in frequency, while reducing diameter moves these modes down. Extender diameter has very little effect on longitudinal modes (e.g. the 23 kHz design frequency), therefore modifying extender diameter may be a useful design tool to make conflicting errant modes not powered in device operation. As the second extender or third horn member consists of a step up and step down, the net effect on gain (e.g. stroke) may be minimal.

It may be noted, the predicate CUSA Excel Laparoscopic surgical tip was limited in surgical tip amplitude, and fragmentation power of tenacious tissue goes with amplitude squared. As laparoscopic surgery has evolved, increasingly diseased tissue is addressed, such as cirrhotic liver tissue, or that affected by chemotherapy. The standard and extended laparoscopic surgical tips have about 15% greater surgical tip amplitude and about 30% greater fragmentation power. The range of surgery can be extended to more tenacious tissue.

In some embodiments, the extended laparoscopic tip can be doubled (e.g. two third horn members between the first and second horn members) and/or triple extended (e.g. three third horn members between the first and second horn members) to allow a very long surgical tip appropriate for seamless integration with a robotic manipulator. Double and/or triple extended tips combined with the flue 20 (e.g. longer or lengthened flue/base body, with the connectors 70, molded portions 40/60, base body 55) may create a surgical tip and flue system long enough to be interfaced with at least one robot during one or more applications. Implementations are not limited to doubled or triple embodiments.

In some implementations, the ultrasonic horn 14 may include one or more third horn members 14c interconnected between the first horn member 14a and the second horn member 14b. The surgical tip 14 (e.g. first and second horn members) can be extended in length L using a half-wave extender or third horn member 14c, without substantially changing the basic design. A titanium horn 14 has a wavelength of the frequency of resonance divided by acoustic velocity, and a half-wavelength is again a half of this length. For illustration, the third horn member 14c of the present extended diameter has a half-wavelength of about 107 mm. An extender or third horn member 14c of this diameter of about 107 mm can be added, for example at an antinode via a threaded attachment/coupling 18. The threaded couplings may be positioned adjacent the antinodes. This extends the standing wave through a node of local maximum of stress and then to another antinode. The resonant frequency and standing wave may not be too greatly impacted with an extender or third horn member. In some applications, a double or two third horn members and/or a triple or three horn members may be added and the surgical tip may be functional in resonance and ability to fragment tissue. The horn members 14a-14c, if used, may be "over torqued" such that the surgical tip 14 may not be practically disassemble in the operating room. For example, one third horn as shown in FIGS. 4D-4F may be added and fastened pneumatically with specialized equipment.

In some implementations, one or more kits may be used for one or more applications. For example, a kit may have one or more flues 20 of varying lengths (e.g. first length, second length, third length, etc.) and/or one or more horns 14 of varying lengths (e.g. first length, second length, third length, etc.).

While several embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific embodiments described herein. It is, therefore, to be understood that the foregoing embodiments are presented by way of example only and that, within the scope of the appended claims, inventive embodiments may be practiced otherwise than as specifically described and claimed. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

The scope of the invention is defined in the claims.

## Claims

1. A flue (20) for use with an ultrasonic horn (14) comprising:
a base (50) having a first connector (72) and a second connector (74) interconnected by a base body (55), and wherein the base body (55) includes a first end and an opposing second end, wherein the base body (55) is of a constant diameter, wherein the first end of the base body (55) includes the first connector (72) and the second end of the base body (55) includes the second connector (74);
wherein the first connector (72) includes a first overmold portion (40) adapted to engage a nosecone (32), wherein a through opening (44) extends through the first overmold portion (40) and wherein the first overmold portion (40) defines an irrigation port (42) and corresponding flue opening (21); and
wherein the second connector (74) includes a second overmold portion (60).

2. The flue (20) of claim 1 wherein at least one of the first connector (72) and the second connector (74) is secured to the base body (55) by at least one of a plastic weld, an adhesive bond, and an adhesive seal.

3. The flue (20) of claim 2 wherein the at least one of the first connector (72) and the second connector (74) is secured to the base body (55) by the plastic weld and the adhesive bond.

4. The flue (20) of claim 3 wherein the at least one of the first connector (72) and the second connector (74) is secured to the base body (55) by the adhesive seal.

5. The flue (20) of claim 1 wherein at least one of the first connector (72) and the second connector (74) includes one or more ribs, and wherein the one or more ribs are overmolded by the first overmold portion (40) and the second overmold portion (60), respectively.

6. The flue (20) of claim 1 wherein the irrigation port (42) is defined in combination with the first connector (72).

7. The flue (20) of claim 1 wherein the second overmold portion (60) includes a through opening (64) narrowing away from the second connector (74).

8. An ultrasonic surgical apparatus (10) for fragmenting tissue and removing fragmented tissue, comprising:
a surgical handpiece (12) comprising a housing (15), a nosecone (32) attached to the housing (15), a flue (20) according to claim 1 attached to the nosecone (32), and a transducer mounted within the housing (15);
a surgical tip connected to the transducer via an internal horn;
an irrigation system connected to the handpiece (12) for supplying irrigation fluid adjacent the surgical site for suspending fragmented tissue;
an aspirating system connected to the handpiece (12) for aspirating fluid and tissue fragmented at the surgical site; and
wherein the flue (20) having a first end and an opposing second end, the first overmold portion (40) defining the first end, and the second overmold portion (60) defining the second end.

9. The apparatus (10) of claim 8 at least one of the first connector (72) and the second connector (74) includes one or more ribs engaging the first overmold portion (40) and the second overmold portion (60), respectively.

10. The apparatus (10) of claim 8 wherein at least one of the first connector (72) and the second connector (74) is secured to the first end and the second end of the base body (55), respectively, by at least one of a plastic weld, an adhesive bond, and an adhesive seal.

11. The apparatus (10) of claim 10 wherein the at least one of the first connector (72) and the second connector (74) is secured to the first end and the second end of the base (50) body (55), respectively, by the plastic weld and the adhesive bond.

12. The apparatus (10) of claim 11 wherein the at least one of the first connector (72) and the second connector (74) is secured to the first end and the second end of the base body (55), respectively, by the adhesive seal.

13. The apparatus (10) of claim 8 wherein the first connector (72) and the second connector (74) are made of a material different from the first overmold portion (40) and the second overmold portion (60).

14. The apparatus (10) of claim 8 wherein the base body (55) is made of a material different from the first and second overmold portions.

## Patentansprüche

1. Rohr (20) zur Verwendung mit einem Ultraschallhorn (14), umfassend:
eine Basis (50), die einen ersten Verbinder (72) und einen zweiten Verbinder (74) aufweist, die durch einen Basiskörper (55) untereinander verbunden sind, und wobei der Basiskörper (55) ein erstes Ende und ein gegenüberliegendes zweites Ende einschließt, wobei der Basiskörper (55) einen konstanten Durchmesser besitzt, wobei das erste Ende des Basiskörpers (55) den ersten Verbinder (72) einschließt und das zweite Ende des Basiskörpers (55) den zweiten Verbinder (74) einschließt;
wobei der erste Verbinder (72) einen ersten Umspritzabschnitt (40) einschließt, der so angepasst ist, dass er in einen Nasenkegel (32) eingreift, wobei sich eine Durchgangsöffnung (44) durch den ersten Umspritzabschnitt (40) erstreckt, und wobei der erste Umspritzabschnitt (40) einen Spülanschluss (42) und eine entsprechende Rohröffnung (21) definiert; und
wobei der zweite Verbinder (74) einen zweiten Umspritzabschnitt (60) einschließt.

2. Rohr (20) nach Anspruch 1, wobei mindestens einer des ersten Verbinders (72) und des zweiten Verbinders (74) am Basiskörper (55) durch mindestens eines von einer Kunststoffschweißung, einer Klebeverbindung und einer Klebedichtung befestigt ist.

3. Rohr (20) nach Anspruch 2, wobei der mindestens eine des ersten Verbinders (72) und des zweiten Verbinders (74) am Basiskörper (55) durch die Kunststoffschweißung und die Klebeverbindung befestigt ist.

4. Rohr (20) nach Anspruch 3, wobei der mindestens eine des ersten Verbinders (72) und des zweiten Verbinders (74) am Basiskörper (55) durch die Klebedichtung befestigt ist.

5. Rohr (20) nach Anspruch 1, wobei mindestens einer des ersten Verbinders (72) und des zweiten Verbinders (74) eine oder mehrere Rippen einschließt, und wobei die eine oder die mehrere Rippen jeweils durch den ersten Umspritzabschnitt (40) und den zweiten Umspritzabschnitt (60) umspritzt sind.

6. Rohr (20) nach Anspruch 1, wobei der Spülanschluss (42) in Kombination mit dem ersten Verbinder (72) definiert ist.

7. Rohr (20) nach Anspruch 1, wobei der zweite Umspritzabschnitt (60) eine Durchgangsöffnung (64) einschließt, die sich von dem zweiten Verbinder (74) weg verschmälert.

8. Chirurgische Ultraschalleinrichtung (10) zum Fragmentieren von Gewebe und Entfernen von fragmentiertem Gewebe, umfassend:
ein chirurgisches Handstück (12), das ein Gehäuse (15), einen Nasenkegel (32), der an dem Gehäuse (15) angebracht ist, ein Rohr (20) nach Anspruch 1, das an dem Nasenkegel (32) angebracht ist, und einen Wandler, der innerhalb des Gehäuses (15) montiert ist, umfasst;
eine chirurgische Spitze, die mit dem Wandler über ein internes Horn verbunden ist;
ein Spülsystem, das zum Zuführen von Spülfluid angrenzend zu der chirurgischen Stelle zum Suspendieren von fragmentiertem Gewebe mit dem Handstück (12) verbunden ist;
ein Aspirationssystem, das zum Aspirieren von Fluid und Gewebe, das an der chirurgischen Stelle fragmentiert wurde, mit dem Handstück (12) verbunden ist; und
wobei das Rohr (20) ein erstes Ende und ein gegenüberliegendes zweites Ende aufweist, wobei der erste Umspritzabschnitt (40) das erste Ende definiert und der zweite Umspritzabschnitt (60) das zweite Ende definiert.

9. Einrichtung (10) nach Anspruch 8, wobei mindestens einer des ersten Verbinders (72) und des zweiten Verbinders (74) eine oder mehrere Rippen einschließt, die jeweils in den ersten Umspritzabschnitt (40) und den zweiten Umspritzabschnitt (60) greifen.

10. Einrichtung (10) nach Anspruch 8, wobei mindestens einer des ersten Verbinders (72) und des zweiten Verbinders (74) jeweils am ersten Ende und am zweiten Ende des Basiskörpers (55) durch mindestens eines von einer Kunststoffschweißung, einer Klebeverbindung und einer Klebedichtung befestigt ist.

11. Einrichtung (10) nach Anspruch 10, wobei der mindestens eine des ersten Verbinders (72) und des zweiten Verbinders (74) jeweils am ersten Ende und zweiten Ende des Basis-(50)-Körpers (55) durch die Kunststoffschweißung und die Klebeverbindung befestigt ist.

12. Einrichtung (10) nach Anspruch 11, wobei der mindestens eine des ersten Verbinders (72) und des zweiten Verbinders (74) jeweils am ersten Ende und am zweiten Ende des Basiskörpers (55) durch die Klebedichtung befestigt ist.

13. Einrichtung (10) nach Anspruch 8, wobei der erste Verbinder (72) und der zweite Verbinder (74) aus einem anderen Material gefertigt sind als der erste Umspritzabschnitt (40) und der zweite Umspritzabschnitt (60).

14. Einrichtung (10) nach Anspruch 8, wobei der Basiskörper (55) aus einem anderen Material gefertigt ist als der erste und zweite Umspritzabschnitt.

## Revendications

1. Gaine (20) pour une utilisation avec un cornet à ultrasons (14) comprenant :
une base (50) présentant un premier connecteur (72) et un deuxième connecteur (74) interconnectés par un corps de base (55), et dans laquelle le corps de base (55) inclut une première extrémité et une deuxième extrémité opposée, dans laquelle le corps de base (55) est de diamètre constant, dans laquelle la première extrémité du corps de base (55) inclut le premier connecteur (72) et la deuxième extrémité du corps de base (55) inclut le deuxième connecteur (74) ;
dans laquelle le premier connecteur (72) inclut une première portion de surmoulage (40) conçue pour venir en prise avec un cône nasal (32), dans laquelle une ouverture traversante (44) s'étend à travers la première portion de surmoulage (40) et dans laquelle la première portion de surmoulage (40) définit un orifice d'irrigation (42) et une ouverture de gaine (21) correspondante ; et
dans laquelle le deuxième connecteur (74) inclut une deuxième portion de surmoulage (60).

2. Gaine (20) selon la revendication 1, dans laquelle au moins l'un parmi le premier connecteur (72) et le deuxième connecteur (74) est fixé au corps de base (55) par au moins l'un parmi une soudure plastique, une liaison adhésive et un joint adhésif.

3. Gaine (20) selon la revendication 2, dans laquelle l'au moins un parmi le premier connecteur (72) et le deuxième connecteur (74) est fixé au corps de base (55) par la soudure plastique et la liaison adhésive.

4. Gaine (20) selon la revendication 3, dans laquelle l'au moins un parmi le premier connecteur (72) et le deuxième connecteur (74) est fixé au corps de base (55) par le joint adhésif.

5. Gaine (20) selon la revendication 1, dans laquelle au moins un parmi le premier connecteur (72) et le deuxième connecteur (74) inclut une ou plusieurs nervures, et dans laquelle les une ou plusieurs nervures sont surmoulées par la première portion de surmoulage (40) et la deuxième portion de surmoulage (60), respectivement.

6. Gaine (20) selon la revendication 1, dans laquelle l'orifice d'irrigation (42) est défini en combinaison avec le premier connecteur (72).

7. Gaine (20) selon la revendication 1, dans laquelle la deuxième portion de surmoulage (60) inclut une ouverture traversante (64) allant en rétrécissant depuis le deuxième connecteur (74).

8. Appareil chirurgical à ultrasons (10) pour fragmenter des tissus et retirer des tissus fragmentés, comprenant :
une pièce à main chirurgicale (12) comprenant un boîtier (15), un cône nasal (32) fixé au boîtier (15), une gaine (20) selon la revendication 1 fixée au cône nasal (32), et un transducteur monté à l'intérieur du boîtier (15) ;
une pointe chirurgicale reliée au transducteur via un cornet interne ;
un système d'irrigation relié à la pièce à main (12) pour fournir un fluide d'irrigation adjacent au site chirurgical pour suspendre des tissus fragmentés ;
un système d'aspiration relié à la pièce à main (12) pour aspirer le fluide et les tissus fragmentés au niveau du site chirurgical ; et
dans lequel la gaine (20) présentant une première extrémité et une deuxième extrémité opposée, la première portion de surmoulage (40) définissant la première extrémité et la deuxième portion de surmoulage (60) définissant la deuxième extrémité.

9. Appareil (10) selon la revendication 8 au moins un parmi le premier connecteur (72) et le deuxième connecteur (74) inclut une ou plusieurs nervures venant en prise avec la première portion de surmoulage (40) et la deuxième portion de surmoulage (60), respectivement.

10. Appareil (10) selon la revendication 8 dans lequel au moins l'un parmi le premier connecteur (72) et le deuxième connecteur (74) est fixé à la première extrémité et à la deuxième extrémité du corps de base (55), respectivement, par au moins l'un parmi une soudure plastique, une liaison adhésive et un joint adhésif.

11. Appareil (10) selon la revendication 10 dans lequel l'au moins un parmi le premier connecteur (72) et le deuxième connecteur (74) est fixé à la première extrémité et à la deuxième extrémité du corps (55) de base (50), respectivement, par la soudure plastique et la liaison adhésive.

12. Appareil (10) selon la revendication 11 dans lequel l'au moins l'un parmi le premier connecteur (72) et le deuxième connecteur (74) est fixé à la première extrémité et à la deuxième extrémité du corps de base (55), respectivement, par le joint adhésif.

13. Appareil (10) selon la revendication 8 dans lequel le premier connecteur (72) et le deuxième connecteur (74) sont constitués d'un matériau différent de la première portion de surmoulage (40) et de la deuxième portion de surmoulage (60).

14. Appareil (10) selon la revendication 8 dans lequel le corps de base (55) est constitué d'un matériau différent des première et deuxième portions de surmoulage.
